# EUROPEAN PATENT APPLICATION

(11) **EP 1 892 289 A2**
(43) Date of publication of application: **27.02.2008**
(21) Application number: 07018107.8
(22) Date of filing: 08.11.2002
(51) Int. Cl.: C12N 5/00, C12N 5/02, C12N 5/06, A01N 63/00, A01N 65/00, A01N 43/04, A61K 31/70

(54) **Adipose tissue-derived stromal cells for the repair of corneal and intra-orbital defects and uses thereof**

(30) Priority: 09.11.2001 US 347605
(62) Divisional of application: 02795611.9
(71) Applicant: Artecel Sciences, Inc., Durham, NC 27701 (US)
(72) Inventor: The designation of the inventor has not yet been filed
(74) Representative: Grünecker, Kinkeldey, Stockmair & Schwanhäusser Anwaltssozietät

(57) **Abstract**

The invention provides compositions and methods for the use of adipose tissue-derived stromal cells for the treatment and repair of any ocular-associated tissue defect, including but not limited to those secondary to trauma, metabolic disease, inborn errors, or surgery. The adipose tissue-derived stromal cells are cultured in the undifferentiated, differentiated or adipocyte state either alone or in the presence of biocompatible material. The resulting materials are employed surgically to correct a variety of correct intra-ocular defects.

## Description

### CROSS REFERENCE TO RELATED APPLICATION

This application claims priority to U.S. Serial No. 60/347,605 filed on November 9,2001.

### FIELD OF INVENTION

The invention provides isolated adipose tissue-derived stromal cells induced to express at least one characteristic of an intra-ocular stromal cell. Methods for the repair of corneal and intra-orbital defects of the eye with differentiated or undifferentiated adipose-derived stromal cells are also provided.

### BACKGROUND OF INVENTION

The eye is a complex organ composed of a multitude of tissue types. Three different coats comprise the eye; from external to internal: the sclera and cornea, the choroids, and the retina. Within the three coats are the refractive media: the aqueous humor, the crystalline lens, and the vitreous humor. Stromal cells are an integral component of each layer of the eye and share important regulatory relationships with the neighboring epithelial, endothelial, neuronal, and inflammatory cells. A number of conditions affect the eye and its function. For example, disorders of the cornea include so-called primary and secondary diseases (Kruse & Volcker, 1997, Curr. Opin. Opthalmol. 8(4):46-54). Primary diseases of the eye, include, but are not limited to: aniridia or absence of the iris; eythrokeratodermia or a reddening and hyperkeratosis of the skin; and keratitis with multiple endocrine deficiencies. Secondary diseases of the eye, include, but are not limited to: chemical injuries; thermal injuries; contact lens keratopathy; repetitive limbal surgeries, and band keratopathy, a degenerative condition in which a gray band develops from the limbus into the cornea.

Many of these disorders involve the self-renewing stem cells of the cornea, which reside in the basal layer of the limbus, lying between the cornea and conjunctiva (Kruse & Volcker, 1997, Curr. Opin. Opthalmol. 8(4):46-54). Limbal insufficiency or stem cell dysfunction results in symptoms that include decreased vision, photophobia, inflammation, edema, and spasm of the muscles controlling the eyelid. In some cases, this can be corrected by transplantation of healthy limbal tissue to diseased areas of the cornea, as in cases of a discretely localized chemical bum. A variety of evidence indicates that the interaction between the corneal stem cells and their underlying stroma is critical for normal function.

The two adjacent cell types display specific autocrine and paracrine pathways. These pathways are mediated by cytokines released by stromal cells, including but not limited to, transforming growth factor β1 and β2 (TGFβ), insulin like growth factor (IGF), basic fibroblast growth factor (bFGF), hepatic growth factor (HGF), and keratinocyte growth factor (KGF). Receptors for each of these are synthesized by the corneal epithelial cells. Likewise, the corneal epithelial cells synthesize TGFβ1 and β2, IGF, bFGF, and vascular endothelial growth factor (VEGF). Except for the VEGF receptor, the stromal cells express receptors for all of these cytokines as well as platelet derived growth factor (PDGF) and epidermal growth factor (EGF) (Kruse & Volcker, 1997, Curr. Opin. Opthalmol. 8(4):46-54).

The cornea plays a critical role in the maintenance of normal vision by refracting light onto the lens and retina. This requires continuous self renewal of the corneal epithelial cells to protect the deeper layers of the tissue from infection (Lu et al 2001, Exp Biol Med 226(7):653-64). Surgical procedures, such as photorefractive keratectomy or laser in situ keratomileusis, involve the removal of a portion of the cornea. These procedures are performed to treat forms of myopia or nearsightedness (Wilson et al 2001, Arch Ophthalmol: 119(6): 889-96).

The repair following these procedures requires stromal contraction accompanied by epithelial stratification (Taliana et al 2001, Invest Ophthalmol Vis Sci; 42(1):81-9). During the healing process, many patients experience a postoperative anterior "stromal haze", characterized in animal models by the appearance of myofibroblastic cells expressing alpha smooth muscle actin (α-SMA) (Nakamura et al 2001, Br J Ophthalmol; 85(2):209-13). These myofibroblastic cells are induced in culture by TGFβ and blocked in vivo by anti-TGFβ antibodies (Jester et al 1997, Cornea; 16(2):177-87; Jester et al 1999, Prog Retin Eye Res; 18-(3):311-56). Likewise, there is evidence that direct interactions between the epithelial cells and adjacent stroma can induce stromal differentiation of myofibroblasts. This is further influenced by the presence of amniotic membranes (Choi & Tseng 2001, Cornea; 20(2):197-204). The extent of "stromal haze" in animal models corresponds to the depth of damage to the corneal stroma during the photorefractive keratectomy (Moller-Pedersen et al 1998, Cornea; 17(6):627-39). In patients, the degree of corneal haze is the same, independent of the technique for epithelial cell removal (mechanical or laser based) (Lee et al 2001, Ophthalmology; 108(1):112-20).

A variety of biomaterials have been used to treat and repair corneal and ocular defects and injuries. The corneal extracellular matrix is rich in collagen and glycosaminoglycans (Robert et al 2001, Pathol Biol (Paris); 49(4):353-63). The glycosaminoglycan, hyaluronan, has been found to improve corneal epithelial wound healing in rat and rabbit models, as assessed by evaluation of the stromal and endothelial layers (Nakamura et al 1997, Exp Eye Res; 64(6):1043-50; Chung et al 1999, Ophthalmic Res; 31(6):432-9). Tseng and others have pioneered the use of amniotic membrane in the treatment of a variety of ocular disorders (US Patent No. 6,152,142). The amniotic membrane is polarized, with a 'stromal' side and a 'basement membrane' side. The stromal side contains collagens I and III and fibronectin with a basal lamina distribution of collagen type IV, laminin and heparin sulfate proteoglycan. The basement membrane side of the amniotic membrane supports epithelial cell growth, while the stromal side supports the growth of fibroblasts in a manner similar to collagen. The amniotic membrane is isolated from the human placenta, cryopreserved, and then used for the surgical repair of intra-ocular disorders.

The mechanism of action of the amniotic membrane remains incompletely understood. However, there is *in vitro* evidence that the presence of amniotic membrane in culture suppresses the expression of TGFβ by fibroblasts (Lee et al 2000, Curr Eye Res; 20(4):325-334) and interleukin 1 α and interleukin 1β by epithelial cells (Solomon et al 2001, Br J Ophthalmol; 85(4): 444-449).

The amniotic membrane has been used successfully to treat a wide range of corneal and ocular defects. For example, deep corneal and scleral ulcers have been treated by the use of multi-layers of the amniotic membrane to fill stromal layer, basement membranes, and as a wound cover (Hanada et at 2001, Am J Opthalmol; 131(3):324-31). Amniotic membrane was found to reduce stromal inflammation and ulceration in HIV-1 keratitis, an immune mediated disease (Heiligenhaus et at 2001, Invest Ophthalmol Vis Sci; 42(9):1969-1974). Severe neurotrophic corneal ulcers also have been treated with amniotic membranes (Chen et al 2000, Br J Ophthalmol; 84(8):826-833). Amniotic membrane restored the corneal and conjunctival surfaces and reduced limbal stromal inflammation resulting from acute chemical or thermal bums (Meller et al 2000, Ophthalmology;107(5): 980-989). Amniotic membrane was used as an alternative to limbal autograft or allograft in patients with partial limbal stem cell deficiency (Anderson et al 2001, Br J Ophthalmol; 85(5):567-575). Amniotic membranes have also been used in surgical treatment of pterygia, a wing-like fold of membrane extending from the conjunctiva to the cornea, with attachments to the sclera (Solomon et al 2001, Ophthalmology: 108(3):449-460). Amniotic membranes were used to treat late onset glaucoma filtering bed leaks as an alternative to conjunctiva with success (Budenz et al 2000, Am J Ophthalmol; 130(5): 580-588; Barton et al 2001, Invest Ophthalmol Vis Sci; 42(8):1762-1768) as well as to improve recovery of a stable corneal epithelium and reduce ocular pain when used in the surgical treatment of band keratopathy, the deposition of calcium in the corneal basement membrane secondary to sarcoidosis, chronic uveitis and other causes (Anderson et al 2001, Cornea; 20(4): 354-361).

Because of the difficulties associated with the procurement of amniotic materials, researchers have been looking for alternative sources of stromal material for repair of ocular or corneal defects.

EP 93830229.6 to Cancedda et al discloses methods to culture already differentiated ocular epithelial cells in vitro to be used for subsequent transplantation for the treatment of corneal defects. The source of the epithelial cells is autologous biopsy material from the healthy eye. Thus a patient serves as both donor and recipient.

US Patent No. 6,117,675 to van der Kooy et al discloses stem cells isolated from the retina of mammals that are differentiated in vitro into retinal pigment epithelial cells that are transplanted into an individual suffering from diseases of the retina. The source of the retinal stem cells is embryonic or early post-natal tissue.

US Patent No. 5,912,178 to Wille, Jr. discloses media and methods for the in vitro formation of various types of histologically complete human epithelium including epidermis, cornea, gingival and ureter tissues. The resulting epithelium is grown in vitro from isolated animal epithelia cells or basal stem cells isolated from epithelial tissue.

US Patent No. 5,942,487 to Ogawa et al discloses a composition comprising a stem cell factor that can be applied to diseased corneal tissue to stimulate corneal growth. The inventors hypothesize that corneal stem cells are stimulated to migrate and grow.

U.S. Patent No. 5,863,531 to Advanced Tissue Sciences, Inc. discloses a tubular living stromal tissue prepared in vitro, comprising stromal cells and connective tissue proteins naturally secreted by the stromal cells attached to and substantially enveloping a three-dimensional tubular framework composed of a biocompatible, non-living material having interstitial spaces bridged by the stromal cells.

It is an object of the invention to provide a cell, material and method to assist in the repair of ocular, including corneal, defects.

### SUMMARY OF THE INVENTION

The present invention provides an adipose-derived stromal or stem cell that has been differentiated to possess at least one genotypic or phenotypic characteristic of an intraocular or ocular stromal cell. This cell can be used for the therapeutic treatment of degenerative or other diseases of the eye.

The present invention also provides methods and compositions for the use and culture of adipose tissue-derived stromal cells to treat intra-ocular or ocular defects of any etiology.

In another aspect of the invention, methods and compositions for consistent qualitative and quantitative induction of stromal cells derived from subcutaneous, mammary, gonadal, omental or any other adipose tissue sites to express at least one genotypic or phenotypic characteristic of intra-ocular or ocular stromal cells is provided.

In another aspect of the invention, isolated adipose tissue-derived cells are induced to differentiate into a cell that expresses at least one characteristic of an intra-ocular stromal cell comprising the step of: contacting an isolated adipose tissue-derived stromal cell with an ocular inducing substance. This substance is in a chemically defined cell culture medium and includes growth factors, cytokines, chemical agents, and/or hormones at concentrations sufficient to induce isolated adipose tissue-derived stromal cells to express at least one ocular cell marker.

In another aspect of the present invention, adipose tissue-derived stromal cells are genetically engineered to express proteins that can modify the cell phenotype and induce pathways that facilitate the recovery and repair of any intra-ocular defect. This is accomplished by exposing the cell to a gene transfer vector comprising a nucleic acid including a transgene under suitable conditions such that the desired nucleic acid is introduced into the cell. Alternatively, the cell is treated with, and allowed to incorporate, a naked nucleic acid.

The invention further provides for a method of treating a degenerative eye condition in a host that includes administering adipose-derived stromal cells than have been induced to express a characteristic of a desired ocular cell. A distinct advantage of the invention is that the adipose tissue-derived stromal cells can be isolated directly from the host (autologous transplantation), or can be donated by another host (allogenic transplantation).

In another aspect of the invention, adipose tissue-derived stromal cells are cultured in their undifferentiated or differentiated state in or on a three dimensional matrix comprised of a natural or synthetic biocompatible material to be used to surgically repair sites of intra-ocular stromal ulceration.

In another aspect of the invention, adipose tissue-derived stromal cells are used directly without differentiation for autologous and allogeneic transplantation of cells to promote successful glaucoma surgeries by correcting bleb leakage. In this embodiment, the adipose-derived stromal cells is administered to the eye, preferably in the desired location, and allowed to differentiate either through (i) co-administration of appropriate cytokines and other biological factors, or (ii) *in vivo* factors already present or induced in the host.

In still another aspect of the present invention, adipose tissue-derived stromal cells are used for autologous and allogeneic transplantation of cells for the treatment of human conditions, including but not limited to, corneal haze induced by photorefractive/therapeutic keratectomy, repair during the "bare sclera" removal of pterygia, surgical treatment of band keratopathy, surgical removal of tumors, lesions, or scar tissue from the conjunctival or corneal surface, among others.

In yet another aspect of the invention, adipose tissue derived stromal cells are transplanted in combination with amniotic membrane tissue into a diseased eye.

In another aspect of the invention, the adipose tissue derived stromal cells are differentiated into cells of adipoblast lineage before transplantation into the diseased eye.

In yet another aspect of the invention, the adipose tissue derived stromal cells are differentiated into cells of adipoblast lineage and transplanted along with amniotic membrane tissue into the diseased eye.

The cells of the invention are used either as a homogenous population of cells or as part of a cell population in which the other cells secrete substances to support the growth or differentiation of the ocular-like cell.

The invention also contemplates a kit for isolating stem or stromal cells from adipose tissue that includes a means for isolating adipose tissue from a patient and a means for separating stem cells from the remainder of the adipose tissue. The kit also includes a medium for differentiating the stem cells, wherein the medium causes the cell to express at least one genotypic or phenotypic characteristic of an ocular cell, or is generally ocularogenic.

The invention further includes compositions and methods for engineering tissue from the adipose-derived cells of the invention. Such compositions include an adipose-derived cell in combination with a biologically compatible lattice structure to allow the tissue to differentiate and expand. Thus in such a way, eye-like tissue or whole organs are manufactured. The biologically compatible lattice can be adipose-derived itself and include any human protein, proteoglycan, glycoprotein, hyaluronin, fibronectin molecule, a hormone, cytokines and growth factors. The lattice can also include or be prepared from polymers of monomers selected from the group consisting of glycolic acid, lactic acid, propyl fumarate, caprolactone, hyaluronan, hyaluronic acid or combinations thereof. Alternatively, the polymeric material can be a protein, polysaccharide, polyhydroxy acid, polyorthoester, polyanhydride, polyphosphazene, synthetic polymer or a combination thereof. The polymeric material can also be a hydrogel formed by cross-linking a polymer suspension having the cells dispersed therein.

Other objects and features of the invention will be more fully apparent from the following disclosure and appended claims.

### BRIEF DESCRIPTION OF THE DRAWINGS

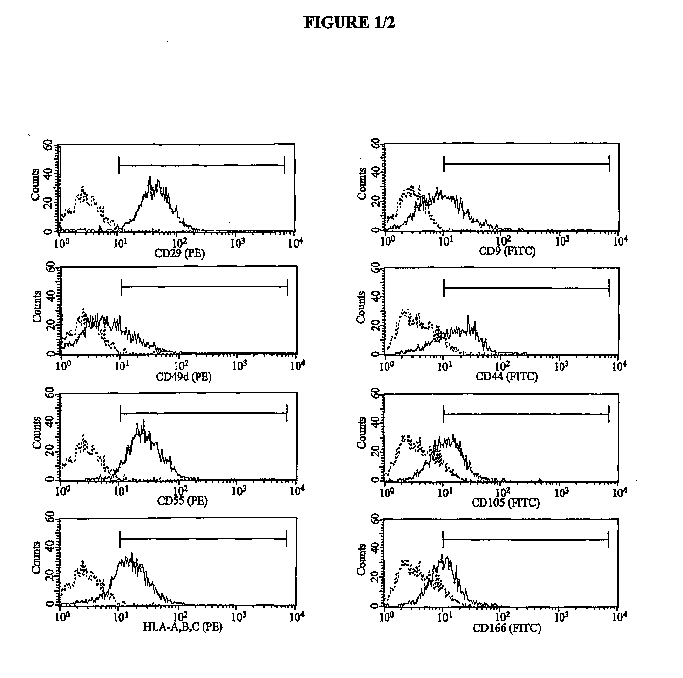

**Figure 1** is a flow cytometric analysis of human adipose-derived stromal cells. Depicted are representative flow cytometric analyses for CD9, CD29 (beta 1-integrin), CD44 (hyaluronate receptor), CD49d (alpha 4 integrin), CD55 (decay accelerating factor), and HLA-ABC (Class 1 histocompatibility antigen). Undifferentiated stromal cells isolated from a single donor were stained with monoclonal antibodies against indicated antigens (solid line, right of each panel); isotype monoclonal control antibody (dotted line, left of each panel). Representative n = 5 donors. Bar indicates fluorescent intensity >99% control.

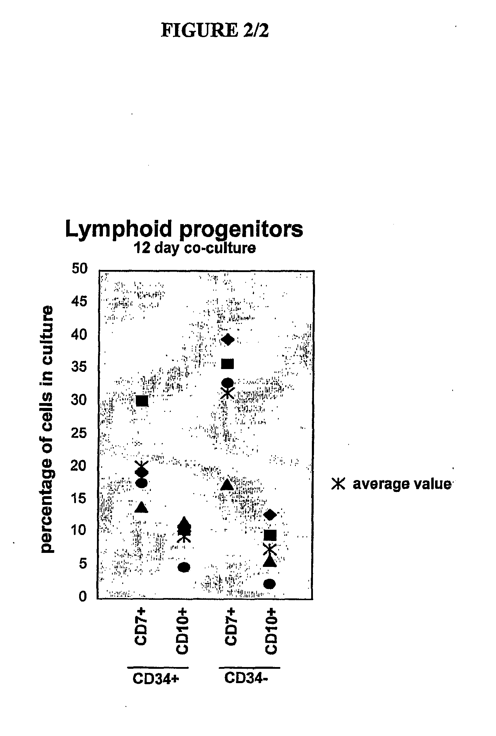

**Figure 2** is a schematic showing lymphoid progenitors in a 12 day co-culture. A significant percentage of both the CD34⁺ and CD34⁻ cells expressed either the CD7 or CD10 antigen (n = 4 stromal donors, n = 2 UCB donors). The individual phenotypes represented the following percentages of the total hematopoietic population: early lymphoid progenitors, 20.2% (CD34⁺ CD7⁺) and 9.5% (CD34⁺ CD10⁺), respectively; NK/T-cell progenitors (CD34⁻ CD7⁺), 31.4%; and B-cell progenitors (CD34- CD10⁺), 7.7%.

### DETAILED DESCRIPTION OF THE INVENTION

The invention is an adipose-derived stromal or stem cell induced to express at least one genotypic or phenotypic characteristic of a cell of ocular origin. More preferably, the cell possesses at least one genotypic or phenotypic characteristic of a corneal epithelial cell. Alternatively, the cell possesses at least one genotypic or phenotypic characteristic of an ocular stromal cell. The cells produced by the methods of invention provide a source of partially or fully differentiated, or undifferentiated functional cells having genotypic or phenotypic characteristics of mature ocular cells for research, transplantation, and development of cellular therapeutic products for the treatment of animal diseases, preferably human diseases, tissue repair or improvement, and the correction of degenerative diseases of the eye, preferably the corneal epithelia. Methods to produce and use such cells are also included.

### I. Definitions

"Conjunctiva" refers to the membrane that lines the eyelid and covers the exposed surface of the sclera.

"Cornea" refers to the transparent structure forming the anterior part of the fibrous tunic of the eye. It consists of five layers, specifically: 1) anterior corneal epithelium, continuous with the conjunctiva; 2) anterior limiting layer (Bowman's membrane); 3) substantia propria, or stromal layer, 4) posterior limiting layer (Descemet's membrane); and 5) endothelium of the anterior chamber or keratoderma.

"Retina" refers to the innermost of the three tunics of the eyeball, surrounding the vitreous body and continuous posteriorly with the optic nerve. It is divided into the pars optica, which rests upon the choroids, the pars ciliaris, which rests upon the ciliary body, and the pars iridica, which rests upon the posterior surface of the iris. Grossly, the retina is composed of an outer, pigmented layer (pars pigmentosa) and an inner, transparent layer (pars nervosa), which together make up the pars optica. The optic part of the eye consists of 9 layers named from within to outward as: 1) internal limiting membrane; 2) nerve fiber layer; 3) layer of ganglion cells; 4) inner plexiform layer; 5) inner nuclear layer; 6) outer plexiform layer; 7) outer nuclear layer; 8) external limiting membrane; and 9) a layer of rods and cones.

"Transforming Growth Factor β" (TGFβ) is a 55 kDa, 391 amino acid (aa) preproprotein that consists of a 23 aa signal sequence, a 256 aa pro-region and a 112 aa mature segment. Prior to secretion, the pro-region is cleaved at an RxxR site with a furin-like protease. This generates a nonglycosylated, 25 kDa, disulfide-linked mature dimer that noncovalently associates with its previously attached disulfide-linked pro-regions to form a "latent complex". This complex is secreted. Activation occurs extracellularly under a variety of conditions most likely via a transmembrane serine/threonine kinase to initiate an intracellular signal cascade mediated by the Smad family of transcription factors.

"Basic Fibroblast Growth Factor" (bFGF), also known as FGF-2, is an 18 kDa, non-glycosylated polypeptide that shows both intracellular and extracellular activity. BFGF is secreted as a monomer. Following secretion, bFGF is sequestered on either cell surface heparin sulfate (HS) or matrix glycosaminoglycans. Although bFGF is secreted as a monomer, cell surface HS seems to dimerize monomeric bFGF in a non-covalent side-to-side configuration that is subsequently capable of dimerizing and activating FGF receptors.

"Platelet Derived Growth Factor" (PDGF) is a 30 kDa homo- or heterodimeric combination of two genetically distinct, but structurally related, polypeptide chains designated A and B. It was originally identified as a platelet derived fibroblast mitogen in serum. Subsequent studies have demonstrated that many cell types secrete PDGF and that the cytokine is a mitogen for cells of the mesodermal lineage (muscle, bone, connective tissue).

"Vascular Endothelial Growth Factor" (VEGF) was first identified as a growth and survival factor for endothelial cells. It induces endothelial cell proliferation and regulates angiogenesis and vasculogenesis. VEGF is a heparin-binding glycoprotein that is secreted as a 45 kDa homodimer. Many cell types, but usually not endothelial cells themselves, secrete VEGF.

"Hepatocyte growth factor" (HGF), also known as scatter factor, is a multifunctional cytokine that promotes mitogenesis, migration, invasion and morphogenesis. HGF-dependent signaling modulates integrin function by promoting aggregation and cell adhesion. HGF/SF-induced effects occur via signaling of the MET tyrosine kinase receptor, following ligand binding.

"Keratinocyte growth factor" (KGF), or FGF-7, is a 28 kDa, single chain, secreted glycoprotein that has a target relatively restricted to epithelium. The molecule is synthesized as a 194 aa precursor that contains a 31 aa signal sequence and a 163 aa mature segment. The mature growth factor binds heparin and its receptor (KGFR) through discrete regions of the molecule. Adult cells that express FGF-7 include fibroblasts, T-cells, smooth muscle cells, and ovarian theca cells. In the embryo, KGF is found at many stages of development throughout the mesenchyme.

"Insulin-like Growth Factors" (IGF), IGF-I and IGF-II, share 50% structural homology to insulin. IGFs act as mitogenic stimulators of cell proliferation as well as suppressors of cellular apoptotic pathways. Under the control of growth hormone (GH), the liver is the primary site of IGF production. IGF-I levels are also influenced by nutrition and developmental stages. Autocrine and paracrine tissue production of IGFs contribute to the levels of IGF available for growth regulation.

"Developmental phenotype" is the potential of a cell to acquire a particular physical phenotype through the process of differentiation.

"Hormone" is any substance that is secreted by a cell and that causes a phenotypic change in the same or another cell upon contact.

"Genotype" is the expression of at least one messenger RNA transcript of a gene associated with a differentiation pathway.

The term "transgenic" is used to describe any animal or any part thereof, including but not restricted to, cells, cultures or tissues which includes exogenous genetic material within its cells. Cells of the invention can have the DNA added to them and these cells can then be used for transplantation or for in vitro production of hormones, cells or tissues.

"Transgene" means any piece of DNA inserted by artifice into a cells that becomes part of the genome of the cell, cell line, tissue or organism (i.e. either stably integrated or as a stable extrachromosomal element) which develops from that cell. Such a transgene may include a gene which is partly or entirely heterologous or foreign to the cell or organism to which the heterologous gene is introduced, or may represent a gene homologous to an endogenous gene of the organism. Included within the definition is a transgene created by the providing of an RNA sequence that is transcribed into DNA and then incorporated into the genome. The term "transgenic" additionally includes any organism or part thereof, including, but not limited to, cells, cell lines, cell cultures or tissues whose genome has been altered by in vitro manipulation or by any transgenic technology.

### II. Adipose-Derived Stem or Stromal Cells

Adipose-derived stem cells or "adipose-derived stromal cells" refer to cells that originate from adipose tissue. By "adipose" is meant any fat tissue. The adipose tissue may be brown or white adipose tissue, derived from subcutaneous, omental/visceral, mammary, gonadal, or other adipose tissue site. Preferably, the adipose is subcutaneous white adipose tissue. Such cells may comprise a primary cell culture or an immortalized cell line. The adipose tissue may be from any organism having fat tissue. Preferably, the adipose tissue is mammalian, most preferably the adipose tissue is human. A convenient source of adipose tissue is from liposuction surgery, however, the source of adipose tissue or the method of isolation of adipose tissue is not critical to the invention.

Adult human extramedullary adipose tissue-derived stromal cells represent a stromal stem cell source that can be harvested routinely with minimal risk or discomfort to the patient. Pathologic evidence suggests that adipose-derived stromal cells are capable of differentiation along multiple lineage pathways. Adipose tissue is readily accessible and abundant in many individuals. Obesity is a condition of epidemic proportions in the United States, where over 50% of adults exceed the recommended BMI based on their height.

It is well documented that adipocytes are a replenishable cell population. Even after surgical removal by liposuction or other procedures, it is common to see a recurrence of adipocytes in an individual over time. This suggests that adipose tissue contains stromal stem cells that are capable of self-renewal.

Adipose tissue offers many practical advantages for tissue engineering applications. First, it is abundant. Second, it is accessible to harvest methods with minimal risk to the patient. Third, it is replenishable. While stromal cells represent less than 0.01% of the bone marrow's nucleated cell population, there are up to 8.6 x 10⁴ stromal cells per gram of adipose tissue (Sen et al 2001, Journal of Cellular Biochemistry 81:312-319). Ex vivo expansion over 2 to 4 weeks yields up to 500 million stromal cells from 0.5 kilograms of adipose tissue. These cells can be used immediately or cryopreserved for future autologous or allogeneic applications.

Adipose derived stromal cells also express a number of adhesion and surface proteins. These include cell surface markers such as CD9; CD29 (integrin beta 1); CD44 (hyaluronate receptor); CD49d,e (integrin alpha 4, 5); CD54 (ICAMI); CD55 (decay accelerating factor); CD105 (endoglin); CD106 (VCAM-1); CD166 (ALCAM) and HLA-ABC (Class I histocompatibility antigen); and cytokines such as interleukins 6, 7, 8, 11; macrophage-colony stimulating factor; GM- colony stimulating factor; granulocyte-colony stimulating factor; leukemia inhibitory factor; stem cell factor and bone morphogenetic protein. Many of these proteins have the potential to serve a hematopoietic supportive function and all of them are shared in common by bone marrow stromal cells.

Methods for the isolation, expansion, and differentiation of human adipose tissue-derived cells have been reported. See for example, Burris et al 1999, Mol Endocrinol 13:410-7; Erickson et al 2002, Biochem Biophys Res Commun. 2002 Jan 18;290(2):763-9; Gronthos et at 2001, Journal of Cellular Physiology, 189:54-63; Halvorsen et al 2001, Metabolism 50:407-413; Halvorsen et al 2001, Tissue Eng. 7(6):729-41; Harp et al 2001, Biochem Biophys Res Commun 281:907-912; Saladin et al 1999, Cell Growth & Diff 10:43-48; Sen et al 2001, Journal of Cellular Biochemistry 81:312-319; Zhou et al 1999, Biotechnol. Techniques 13: 513-517. Adipose tissue-derived stromal cells are obtained from minced human adipose tissue by collagenase digestion and differential centrifugation [Halvorsen et al 2001, Metabolism 50:407-413; Hauner et al 1989, J Clin Invest 84:1663-1670; Rodbell et al 1966, J Biol Chem 241:130-139]. Others have demonstrated that human adipose tissue-derived stromal cells can differentiate along the adipocyte, chondrocyte, and osteoblast lineage pathways [Erickson et al 2002, Biochem Biophys Res Commun. 2002 Jan 18;290(2):763-9; Gronthos et al 2001, Journal of Cellular Physiology, 189:54-63; Halvorsen et al 2001, Metabolism 50:407-413; Halvorsen et al, 2001, Tissue Eng. 2001 Dec;7(6):729-41; Harp et al 2001, Biochem Biophys Res Commun 281:907-912; Saladin et al 1999, Cell Growth & Diff 10:43-48; Sen et al 2001, Journal of Cellular Biochemistry 81:312-319; Zhou et al 1999, Biotechnol. Techniques 13: 513-517; Zuk et al 2001, Tissue Eng. 7: 211-228.

Human adipose tissue-derived adult stromal cells represent an adult stem cell source that can be harvested routinely with minimal risk or discomfort to the patient. They can be expanded ex vivo, differentiated along unique lineage pathways, genetically engineered, and re-introduced into individuals as either autologous or allogeneic transplantation.

WO 00/53795 to the University of Pittsburgh and The Regents of the University of California and US Patent Application No. 2002/0076400 assigned to the University of Pittsburgh, disclose adipose-derived stem cells and lattices substantially free of adipocytes and red blood cells and clonal populations of connective tissue stem cells. The cells can be employed, alone or within biologically-compatible compositions, to generate differentiated tissues and structures, both in vivo and in vitro. Additionally, the cells can be expanded and cultured to produce hormones and to provide conditioned culture media for supporting the growth and expansion of other cell populations. In another aspect, these publications disclose a lipo-derived lattice substantially devoid of cells, which includes extracellular matrix material form adipose tissue. The lattice can be used as a substrate to facilitate the growth and differentiation of cells, whether in vivo or in vitro, into anlagen or mature tissue or structures. Neither publication discloses adipose tissue derived stromal cells that have been induced to express at least one phenotypic or genotypic characteristic of an intra-ocular stromal cell.

U.S. Patent No. 6,391,297 assigned to Artecel Sciences discloses a composition of an isolated human adipose tissue-derived stromal cell that has been differentiated to exhibit at least one characteristic of an osteoblast that can be used in vivo to repair bone and treat bone diseases. This adipose-derived osteoblast-like cell can be optionally genetically modified or combined with a matrix.

U.S. Patent No. 6,426,222 assigned to BioHoldings International discloses methods for inducing osteoblast differentiation from human extramedullary adipose tissue by incubating the adipose tissue cells in a liquid nutrient medium that must contain a glucocorticoid.

WO 00/44882 and U.S. Patent No. 6,153,432 listing Halvorsen et al as inventors, discloses methods and compositions for the differentiation of human preadipocytes isolated from adipose tissue into adipocytes bearing biochemical, genetic, and physiological characteristics similar to that observed in isolated primary adipocytes.

WO 01/62901 and published U.S. Patent Application No. 2001/0033834 to Artecel Sciences discloses isolated adipose tissue-derived stromal cells that have been induced to express at least one phenotypic characteristic of a neuronal, astroglial, hematopoietic progenitor or hepatic cell. In addition, an isolated adipose tissue-derived stromal cell that has been dedifferentiated such that there is an absence of adipocyte phenotypic markers is also disclosed.

U.S. Patent No. 6,429,013 assigned to Artecel Sciences discloses compositions directed to an isolated adipose tissue-derived stromal cell that has been induced to express at lease one characteristic of a chondrocyte. Methods are also disclosed for differentiating these cells.

U.S. Patent No. 6,200,606 to Peterson et al. discloses that precursor cells which have the potential to generate bone or cartilage can be isolated from a variety of hematopoietic and non-hematopoietic tissues including peripheral blood, bone marrow and adipose tissue.

The adipose tissue derive stromal cells useful in the methods of invention are isolated by a variety of methods known to those skilled in the art such as described in WO 00/53795 to the University of Pittsburgh et al. In a preferred method, adipose tissue is isolated from a mammalian subject, preferably a human subject. A preferred source of adipose tissue is omental adipose. In humans, the adipose is typically isolated by liposuction. If the cells of the invention are to be transplanted into a human subject, it is preferable that the adipose tissue be isolated from that same subject so as to provide for an autologous transplant. Alternatively, the transplanted cells are allogeneic.

As a non-limiting example, in one method of isolating adipose tissue derived stromal cells, the adipose tissue is treated with collagenase at concentrations between 0.01 to 0.5%, preferably 0.04 to 0.2%, most preferably 0.1%, trypsin at concentrations between 0.01 to 0.5%, preferably 0.04 to 0.04%, most preferably 0.2%, at temperatures between 25° to 50°C, preferably between 33° to 40°C, most preferably at 37°C, for periods of between 10 minutes to 3 hours, preferably between 30 minutes to 1 hour, most preferably 45 minutes. The cells are passed through a nylon or cheesecloth mesh filter of between 20 microns to 800 microns, more preferably between 40 to 400 microns, most preferably 70 microns. The cells are then subjected to differential centrifugation directly in media or over a Ficoll or Percoll or other particulate gradient. Cells can be centrifuged at speeds of between 100 to 3000X g, more preferably 200 to 1500X g, most preferably at 500X g for periods of between 1 minute to 1 hour, more preferably 2 to 15 minutes, most preferably 5 minutes, at temperatures of between 4° to 50°C, preferably between 20° to 40°C, most preferably at 25°C.

In yet another method of isolating adipose-derived stromal cells a mechanical system such as described in US 5,786,207 to Katz et al is used. A system is employed for introducing an adipose tissue sample into an automated device, subjecting it to a washing phase and a dissociating phase wherein the tissue is agitated and rotated such that the resulting cell suspension is collected into a centrifuge-ready receptacle. In such a way, the adipose-derived cells are isolated from a tissue sample, preserving the cellular integrity of the desired cells.

### III. Inducement of Adinose-Derived Stromal Cells To Exhibit At Least One Characteristic of an Ocular Cell

The invention includes the treatment of an adipose-derived stromal cell to induce it to form a cell that expresses at least one genotypic or phenotypic characteristic of an ocular cell. Non-limiting examples of how to induce the differentiation of adipose-derived stromal cells include: 1) the use of cell media; 2) the use of support cells; 3) direct implantation of the undifferentiated cells into the tissue of a patient; and 4) cellular engineering techniques.

### A) Cell Media Inducement

While the invention is not bound by any theory of operation, it is believed that treatment of the adipose-derived stromal cells with a medium containing a combination of serum, embryonic extracts, amniotic tissue/fluids, purified or recombinant growth factors, cytokines, hormones, and/or chemical agents, in a 2-dimensional or 3-dimensional microenvironment, will induce the desired differentiation.

More specifically, the invention provides a method for differentiating an adipose-derived cell into a cell having a genotypic or phenotypic property of an ocular cell, comprising: plating isolated adipose-derived adult stem cells at a desired density, including but not limited to a density of about 1,000 to about 500,000 cells/cm²; incubating the cells in a chemically defined culture medium comprising at least one compound selected from the group consisting of: growth factor, hormone, cytokine, serum factor, nuclear hormone receptor ligand, or any other defined chemical agent.

In still another aspect of the invention, the differentiated cells of the invention are grown in cultures until evidence of corneal epithelium formation is observed. Media containing the epithelium is then analyzed by standard biochemical analytical techniques known to those skilled in the art for the presence of markers that would be indicative of corneal epithelial function. The layers of corneal epithelium are then engrafted into the host tissue for tissue generation or regeneration purposes.

Base media useful in the methods of the invention include, but are not limited to, Neurobasal^{™} (supplemented with or without, fetal bovine serum or basic fibroblastic growth factor (bFGF)), N2, B27, Minimum Essential Medium Eagle, ADC-1, LPM (Bovine Serum Albumin-free), F10(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM-without serum), Yamane, IMEM-20, Glasgow Modification Eagle Medium (GMEM), Leibovitz L-15 Medium, McCoy's 5A Medium, Medium M199 (M199E-with Earle's sale base), Medium M199 (M199H-with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA-with non essential amino acids), among numerous others, including medium 199, CMRL 1415, CMRL 1969, CMRL 1066, NCTC 135, MB 75261, MAB 8713, DM 145, Williams' G, Neuman & Tytell, Higuchi, MCDB 301, MCDB 202, MCDB 501, MCDB 401, MCDB 411, MDBC 153. A preferred medium for use in the present invention is DMEM. These and other useful media are available from GIBCO, Grand Island, N.Y., USA and Biological Industries, Bet HaEmek, Israel, among others. A number of these media are summarized in Methods in Enzymology, Volume LVIII, "Cell Culture", pp. 62-72, edited by William B. Jakoby and Ira H. Pastan, published by Academic Press, Inc.

Preferred media for culturing corneal epithelial cells are known in the art and include the media described in U.S. Patent No. 5,912,175 to Wille, Jr. Generally, media useful in the methods of the invention will contain fetal serum of bovine or other species origin at a concentration of at least 1% to about 30%, preferably at least about 5% to 15%, most preferably about 10%. Embryonic extract of chicken or other species origin are present at a concentration of about 1% to 30%, preferably at least about 5% to 15%, most preferably about 10%.

Growth factors, cytokines, hormones and other specific factors useful in the invention include, but not limited to, growth hormone, erythropoietin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor, fibroblast growth factor, nerve growth factor, cilary neurotrophic factor, platelet derived growth factor, and bone morphogenetic protein at concentrations of between picogram/ml to milligram/ml levels. For example at such concentrations, the growth factors, cytokines and hormones useful in the methods of the invention are able to induce, up to 100% the formation of blood cells (lymphoid, erythroid, myeloid or platelet lineages) in colony forming unit (CFU) assays. (Moore et al. (1973) J. Natl. Cancer Inst. 50:603-623; Lee et al. (1989) J. Immunol. 142:3875-3883; Medina et al. (2993) J. Exp. Med. 178:1507-1515.

It is further recognized that additional components may be added to the culture medium. Such components include antibiotics, albumin, amino acids, and other components known to the art for the culture of cells. Additionally, components may be added to enhance the differentiation process. By "chemical agents" is meant steroids, retinoids, and other chemical compounds or agents that induce the differentiation of adipose derived stromal cells by at least 25-50% relative to a positive control.

It is recognized that the cell media condition described above yield a cell which expresses at least one genotypic or phenotypic characteristic of a single type of ocular cell. The particular cell types are separated by any means know to those skilled in the art. Particularly useful are means that take advantage of the genotypic or phenotypic characteristics expressed by the differentiated cells. Phenotypic markers of the desired cells as listed below are well known to those of ordinary skill in the art, and copiously published in the literature. Additional phenotypic markers continue to be disclosed or can be identified without undue experimentation. Any of these markers are used to confirm that the adipose-derived adult stem cells have been induced to a differentiated state. Lineage specific phenotypic characteristics include, but are not limited to, cell surface proteins, cytoskeletal proteins, cell morphology, and/or secretory products. For example, differentiated corneal epithelial cells synthesize receptors for transforming growth factor β1 and β2 (TGFβ), insulin like growth factor (IGF), basic fibroblast growth factor (bFGF), hepatic growth factor (HGF), and keratinocyte growth factor (KGF) (Kruse & Volcker, 1997, Curr Opin Opthalmol; 8(4):46-54). One of ordinary skill in the art will recognize that known calorimetric, fluorescent, immunochemical, polymerase chain reaction, chemical or radiochemical methods readily ascertain the presence or absence of a lineage specific marker.

In another embodiment, the invention provides a dedifferentiated, isolated, adipose-derived adult stem cell capable of being induced to express at least one genotypic or phenotypic characteristic of an ocular cell within a culture medium capable of such differentiation. A dedifferentiated adipose-derived adult stem cell is identified by the absence of mature adipocyte markers.

### B) Use of Support Cells To Promote the Differentiation of the Adipose-Derived Stromal Cells

In another embodiment of the invention, support cells are used to promote the differentiation of the adipose-derived stromal cells. Thus, adipose-derived cells of the invention are isolated and cultured within a population of cells, most preferably the population is a defined population. The population of cells is heterogeneous and includes support cells for supplying factors to the cells of the invention. Support cells include other cell types which will promote the differentiation, growth and maintenance of the desired cells. As a non-limiting example, if an adipose-derived stromal cell that expresses at least one genotypic or phenotypic characteristic of a corneal epithelial cell is desired, adipose-derived stromal cells are first isolated by any of the means described above, and grown in culture in the presence of other ocular or non-ocular support cells. In another embodiment, the support cells are derived from primary cultures of these cell types taken from cultured corneal tissue. In yet another embodiment, the support cells are derived from immortalized cell lines. In some embodiments, the support cells are obtained autologously. In other embodiments, the support cells are obtained allogeneically.

It is also contemplated by the present invention that the cells used to support the differentiation of the desired cell can be genetically engineered to be a support cell. The cells are genetically modified to express exogenous genes or to repress the expression of endogenous genes by any method described below or know to those skilled in the art. One method of genetically modifying the cells of the present invention involves exposing the cells to naked nucleic acid fragments (i.e. DNA or RNA) such that the nucleic acids are incorporated into the genome of the cell in a manner that the nucleic acids are expressed within the cell. Alternatively, the cells of the invention are exposed to a gene transfer vector comprising a nucleic acid including a transgene, such that the nucleic acid is introduced into the cell under conditions appropriate for the transgene to be expressed within the cell.

### C) Implantation

In another aspect, the invention provides adipose-derived stromal cells and differentiated cells expressing at least one genotypic or phenotypic characteristic of an ocular cell that are useful in autologous and allogeneic transplantations. Preferably, the subject is mammalian, more preferably, the subject is human.

Thus, in still another aspect, the invention discloses a method for providing differentiated cells capable of expressing at least one a genotypic or phenotypic characteristic of an ocular cell to a subject, comprising:
a) isolating adipose tissue-derived stromal cells;
b) plating and incubating the cells in a medium appropriate for the differentiation of the cells;
c) introducing the differentiated cells into the subject.

In another embodiment of the invention, a method for providing undifferentiated adipose-derived stromal cells to a subject, comprising:
a) isolating adipose tissue-derived stromal cells;
b) introducing the undifferentiated cells into the subject.

It is contemplated in the invention that when undifferentiated adipose-derived stromal cells are introduced into the subject, in one particular embodiment, they are introduced directly into a diseased eye. In yet another aspect of the invention, the undifferentiated adipose-derived stromal cells are introduced along with any of the support cells or media or differentiation factors as described herein that will provide an environment suitable for the in vivo differentiation of the stromal cells. For example, these support cells in one particular embodiment are amniotic cells. In another embodiment, the support cells are derived from primary cultures of these cell types taken from cultured corneal tissue. In yet another embodiment, the support cells are derived from immortalized cell lines. In some embodiments, the support cells are obtained autologously. In other embodiments, the support cells are obtained allogeneically.

In another embodiment, the dedifferentiated adipose-derived cell is provided in combination with a pharmaceutically acceptable carrier for a therapeutic application, including but not limited to tissue repair, regeneration, reconstruction or enhancement. Adipose-derived cells can be cultured by methods disclosed in U.S. Patent No. 6,153,432 (herein incorporated by reference) to dedifferentiate the cells such that the dedifferentiated adult stem cells are then induced to express genotypic or phenotypic characteristics of cells other than adipose tissue derived cells. The dedifferentiated adipose-derived cells can be modified to include a non-endogenous gene sequence for production of a desired protein or peptide. The dedifferentiated adipose-derived cell can, in an alternative embodiment, be administered to a host in a two- or three-dimensional matrix for a desired therapeutic purpose. In one embodiment, the dedifferentiated cell is obtained autologously from the patient's own cells. Alternatively, the dedifferentiated cell is obtained allogeneically.

### D) Genetic Manipulation of the Adipose-Derived Cells of the Invention

Stem cells and their progeny are important targets for gene therapy, where the inserted genes promote the health of the individual and to whom the stem cells are transplanted.

In yet another embodiment, the adipose-tissue derived cell expressing at least one genotypic or phenotypic characteristic of an ocular cell are genetically modified to express exogenous genes or to repress the expression of endogenous genes. The invention provides a method of genetically modifying such cells and populations. One method of genetically modifying the cells of the present invention involves exposing the cells to naked nucleic acid fragments (i.e. DNA or RNA) such that the nucleic acids are incorporated into the genome of the cell such that the nucleic acids are expressed within the cell.

Alternatively, the cells of the invention are exposed to a gene transfer vector comprising a nucleic acid including a transgene, such that the nucleic acid is introduced into the cell under conditions appropriate for the transgene to be expressed within the cell. The transgene can be an expression cassette, including a coding polynucleotide operably linked to a suitable promoter. The coding polynucleotide can encode a protein, or it can encode biologically active RNA (e.g. antisense RNA or a ribozyme). Thus, for example, the coding polynucleotide can encode a gene conferring resistance to a toxin, a hormone, a cytokine, a cell-surface bound intracellular signaling moiety (e.g. cell adhesion molecules, receptors etc), a factor promoting growth or secretion of an endogenous hormone, peptide or other factor, or any other cellular product. Where it is desired to employ gene transfer technology to deliver a given transgene, its sequence will be known.

Within the expression cassette, the coding polypeptide is operably linked to a suitable promoter. Examples of suitable promoters include prokaryotic promoters and viral promoters (e.g. retroviral promoters, herpes virus promoters, cytomegalovirus promoters). Other suitable promoters are eukaryotic promoters such as enhancers, constitutively active promoters, signal specific promoters and tissue specific promoters. It will be well known to those skilled in the art to select the appropriate promoter suitable for driving gene expression in a predefined cellular context. The expression cassette can include more than one coding polynucleotide, and it can include other elements (e.g. poly-A sequences, transcriptional regulatory elements and the like) as desired.

The expression cassette containing the transgene should be incorporated into a genetic vector suitable for delivering the transgene to the cells. Depending on the desired end application, any such vector can be so employed to genetically modify the cells such as plasmids, naked DNA, or viruses. Any method of constructing the desired expression cassette within the vectors used can be employed. These methods are well known in the art and include such methods as direct cloning, homologous recombination and the like. Those skilled in the art will understand that the choice of vector will largely determine the method used to introduce the vector into the cells.

The genetically altered cells can then be introduced into the organism by a variety of methods under conditions for the transgene to be expressed in vivo. Thus, in a preferred embodiment of the invention, the transgene can encode for growth factors such as TGFβ. The cells containing the transgene for TGFβ can then be introduced into the eye of a diseased human or other mammal.

Alternatively, exogenous foreign or homologous nucleic acids are transferred to the cells of the present invention by a variety of other techniques familiar to those skilled in the art. Thus, nucleic acids are transferred by such methods including, but not limited to, electroporation, calcium phosphate, microinjection, lipofection, retro-or other viral or microbial vector or any other means known to those skilled in the art.

### E) Cellular Characterization

By "characterization" of the resulting differentiated cells is intended the identification of surface and intracellular proteins, genes, and/or other markers indicative of the lineage commitment of the stromal cells to a particular terminal differentiated state. These methods include, but are not limited to, (a) detection of cell surface proteins by immunofluorescent methods using protein specific monoclonal antibodies linked using a secondary fluorescent tag, including the use of flow cytometric methods; (b) detection of intracellular proteins by immunofluorescent methods using protein specific monoclonal antibodies linked using a secondary fluorescent tag, including the use of flow cytometric methods; (c) detection of cell genes by polymerase chain reaction, in situ hybridization, and/or northern blot analysis. Terminally differentiated cells may be characterized by the identification of surface and intracellular proteins, genes, and/or other markers indicative of the lineage commitment of the stromal cells to a particular terminal differentiated state. These methods will include, but are not limited to, (a) detection of cell surface proteins by immunofluorescent assays such as flow cytometry or in situ immunostaining of adipose-derived stromal cells surface proteins such as alkaline phosphatase, CD44, CD146, integrin beta 1 or osteopontin (Gronthos et al. 1994 Blood 84:4164-4173), (b) detection of intracellular proteins by immunofluorescent methods such as flow cytometry or in situ immunostaining of adipose tissue-derived stromal cells using specific monoclonal antibodies; (c) detection of the expression of lineage selective mRNAs such as those produced by corneal epithelial cells including TGFβ1 and β2, IGF, bFGF, and vascular endothelial growth factor (VEGF) by methods such as polymerase chain reaction, in situ hybridization, and/or other blot analysis (See Gimble et al. 1989 Blood 74:303-311).

### F) Use of the Cells of the Invention as Therapeutic Agents

Most preferably, the cells and populations of the present invention are employed as therapeutic agents. In one embodiment, the adipose-derived stromal cell is administered to the eye, preferably in the desired location, and allowed to differentiate either through (i) co-administration of appropriate cytokines and other biological factors, or (ii) *in vivo* factors already present or induced in the host. Generally, such methods involve transferring the cells to the desired tissue or depot, either in vitro as a graft prior to implantation or engrafting or in vivo to the animal directly. The cells are transferred to the desired tissue by any method appropriate, which generally will vary according to the tissue type. For example, cells are transferred to a graft by bathing the graft or infusing it with culture medium containing the cells. Alternatively, the cells are seeded on the desired site within the tissue to establish a population. Cells can be transferred to sites in vivo using devices well know to those skilled in the art for example, catheters, trocars, cannulae, or stents seeded with the cells etc.

The differentiated or undifferentiated adipose-derived stromal cells of the invention find use in therapy for a variety of disorders. The cells are used to treat disorders of the cornea including but not limited to: aniridia or absence of the iris; eythrokeratodermia or a reddening and hyperkeratosis of the skin; keratitis with multiple endocrine deficiency; chemical injuries; thermal injuries; contact lens keratopathy; and repetitive limbal surgeries or limbal insufficiency. Additionally, the cells of the invention are used to regenerate the cornea following such surgical procedures as photorefractive keratectomy or laser in situ keratomileusis, both of which involve the removal of a portion of the cornea. Recovery of these procedures often results in a postoperative anterior "stromal haze", characterized in animal models by the appearance of myofibroblastic cells expressing alpha smooth muscle actin (α-SMA) which are prevented by the use of the cells and procedures of the present invention. The disease-state to be treated may be the result of an autoimmune dysfunction or infection by a virus or some other infectious agent.

### IV. Tissue Engineering

The cells described herein can be employed in tissue engineering. The invention provides methods for producing animal matter comprising maintaining the inventive cells under conditions sufficient for them to expand and differentiate to the desired matter. The matter can include, for example a portion of an eye. As such, the cells described herein are used in combination with any known technique of tissue engineering, including but not limited to those technologies described in the following: U.S. Patent Nos. 5,902,741 and 5,863,531 to Advanced Tissue Sciences, Inc.; U.S. Patent No, 6,139,574, Vacanti et al.; U.S. Patent No. 5,759,830, Vacanti et al.; U.S. Patent No. 5,741,685, Vacanti,; U.S. Patent No. 5,736,372, Vacanti et al.; U.S. Patent No. 5,804,178, Vacanti et al.; U.S. Patent No. 5,770, 417, Vacanti et al.; U.S. Patent No. 5,770,193, Vacanti et al.; U.S. Patent No. 5,709,854, Griffith-Cima et al.; U.S. Patent No. 5,516,532, Atala et al.; U.S. Patent No. 5,855,610, Vacanti et al.; U.S. Patent No. 5,041,138, Vacanti et al.; U.S. Patent No. 6,027,744, Vacanti et al.; U.S. Patent No. 6,123,727, Vacanti et al.; U.S. Patent No. 5,536,656, Kemp et al.; U.S. Patent No. 5,144,016, Skjak-Braek et al.; U.S. Patent No. 5,944,754, Vacanti; U.S. Patent No. 5,723,331, Tubo et al.; and U.S. Patent No. 6,143,501, Sittinger et al..

To produce such a structure, the cells and populations are maintained under conditions suitable for them to expand and divide to form the organ. This may be accomplished by transferring them to an animal typically at a site at which the new matter is desired. Thus, the invention can facilitate the regeneration of part of an eye within an animal where the cells are implanted into such tissues.

In still other embodiments, the cells are induced to differentiate and expand into tissue in vitro. As such, the cells are can be administered alone or in a mixture of cells, or alternatively can be cultured on substrates that facilitate formation into three-dimensional structures conducive for tissue development. Thus, for example, the cells can be cultured or seeded on to a bio-compatible lattice, such as one that includes extracellular matrix material, synthetic polymers, cytokines, growth factors, etc. Such a lattice can be molded into desired shapes for facilitating the development of tissue types.

Thus, the invention provides a composition comprising the inventive cells and populations and a biologically compatible lattice. The lattice can be formed from polymeric material, having fibers as a mesh or sponge, typically with spaces on the order of between 100 µm and about 300 µm. Such a structure provides sufficient area on which the cells can grow and proliferate. Desirably, the lattice is biodegradable over time, so that it will be absorbed into the animal matter as it develops. Suitable polymeric or copolymeric lattices are prepared using monomers such as glycolic acid, lactic acid, propyl fumarate, caprolactone, and the like. Other lattices can include proteins, polysaccharides, polyhydroxy acids, polyorthoesters, polyanhydrides, polyphosphozenes, or synthetic polymers, particularly biodegradable polymers, or any combination thereof. Also, the lattice can include hormones, such as growth factors, cytokines, morphogens (e.g. retinoic acid etc), desired extracellular matrix materials (e.g. fibronectin, laminin, collagen etc) or other materials (e.g. DNA, viruses, other cell types etc) as desired.

To form the composition, the cells are introduced into the lattice such that they permeate into interstitial spaces therein. For example, the matrix can be soaked with a solution or suspension containing the cells, or the cells solution or suspension can be infused or injected into the matrix. Preferably, a hydrogel formed by cross-linking of a suspension including the polymer and also having the inventive cells dispersed therein is used. This method of formation permits the cells to be dispersed throughout the lattice, facilitating more even permeation of the lattice with the cells. Of course, the composition also can include mature cells of a desired phenotype or precursors thereof, particularly to potentiate the induction of the inventive cells within the lattice or promote the production of hormones within the lattice.

Those skilled in the art would appreciate that lattices suitable for inclusion into the composition are derived from any suitable source, e.g. matrigel, and can of course include commercial sources for suitable lattices. Another suitable lattice is derived from the acelluar portion of adipose tissue for example adipose tissue extracellular matrix substantially devoid of cells. Typically such adipose-derived lattices include proteins such as proteoglycans, glycoproteins, hyaluronin, fibronectins, collagens and the like, all of which serve as excellent substrates for cell growth. Additionally, such adipose-derived lattices can include hormones, cytokine, growth factors and the like. Those skilled in the art would be aware of methods for isolating such an adipose-derived lattice such as that disclosed in WO 00/53795 to the University of Pittsburgh, incorporated herein by reference.

The cells, populations, lattices and compositions of the invention are used in tissue engineering and regeneration. Thus, the invention pertains to an implantable structure incorporating any of the disclosed inventive features. The exact nature of the implant will vary according to the use desired. The implant can comprise mature tissue or can include immature tissue or the lattice. Thus for example, an implant can comprise a population of the inventive cells that are undergoing differentiation, optionally seeded within a lattice of a suitable size and dimension. Such an implant is injected or engrafted within a host to encourage the generation or regeneration of ocular tissue within the patient.

The adipose-derived lattice is conveniently employed as part of a cell culture kit. Accordingly, the invention provides a kit including the inventive adipose-derived lattice and one or more other components, such as hydrating agents (e.g. water, physiologically-compatible saline solutions, prepared cell culture media, serum or combinations or derivatives thereof), cell culture substrates (e.g. dishes, plates vials etc), cell culture media (whether in liquid or powdered form), antibiotics, hormones and the like. While the kit can include any such ingredients, preferably it includes all ingredients necessary to support the culture and growth of the desired cells upon proper combination. The desired kit can also include cells which are seeded into the lattice as described.

Alternatively, the cells of the invention are differentiated into cells possessing at least one characteristic of a corneal epithelial cell which are expanded in vitro to grow corneal epithelial tissue for subsequent implantation into a patient. The cells are implanted alone or in combination with other tissue such as amniotic membrane tissue.

The present invention now will be described more fully by the following examples. This invention may, however, be embodied in many different forms and should not be construed as limited to the embodiments set forth herein. Rather, these embodiments are provided so that this disclosure will be thorough and complete, and will fully convey the scope of the invention to those skilled in the art.

### EXAMPLES

### Example 1

### In Vitro Inductive Methods

Adipose-derived stem cells are isolated from liposuction waste material as described (Sen et al., 2001, Journal of Cellular Biochemistry 81:312-319). These cells may be continued in culture in the presence of, but not limited to, the following media: Neurobasal^{™} *(InVitrogen)* supplemented with or without fetal bovine serum (FBS), N2, B27 *(InVitrogen),* or basic fibroblastic growth factor (bFGF). Modulation of glucose levels in the media will also be performed. Cells are seeded at various densities and fed at intervals of every 3-6 days. Most preferably, they are seeded at a density of about 1000 to about 500,000 cells/cm².

During the culture period, conditioned media are analyzed using commercially available radio-immunoassays or enzyme-linked immunosorbent assays for biochemical markers and the expression of phenotypic markers associated with the ocular stromal cells or corneal epithelial cells. Immunohistochemical (IHC) analyses are performed using antibodies against (but not limited to) any of the above described phenotypic markers.

### Example 2

### Use of Adipose-Derived Cells With a Biocompatible Membrane

Approaches to the use of adipose tissue-derived stromal cells in their undifferentiated or adipocyte differentiated state in a composition with a biocompatible material for transplantation to repair an intra-ocular lesion are provided. Surgical patients undergo phototherapeutic keratectomy using a 193 nm excimer beam generated within a VISX Star S2 Excimer Laser (VISX, Inc., Santa Clara, CA) and delivered at 10 Hz with a fluence of 160 mJ/cm² as described in Lee et al (2001, Journal of Cellular Biochemistry 81:312-319). A standardized, central 6-mm diameter laser photoablation is performed with a 45 µm depth setting for epithelial debridement and an appropriate setting for stromal ablation. After PRK, either undifferentiated or adipocyte differentiated adipose tissue-derived adult stem cells are introduced into the ablated area in a composition with a biocompatible material. Prior to introduction, the adipose-derived cells are cultured in a directional manner on the surface of a biocompatible membrane, including but not limited to, amniotic membrane, swine intestinal submucosa, Ampligraft^{™}, Dermagraft^{™}, or a similar product. The adipose-derived cells are cultured as either undifferentiated fibroblast-like cells or induced to undergo adipocyte differentiation according to the methods described in Halvorsen et al (2001, Metabolism 50:407-413). The cell/biomaterial composite is cut to fit the defect size. The adipose-derived cell surface is placed adjacent to the denuded area of cornea. The composite of the cells/biomaterial is sutured into place using 10-0 nylon using interrupted or continuous bites in the cornea and limbus; any excess composite is trimmed away (Anderson et al 2001, Br J Ophthalmol; 85(5):567-575). Following the surgery, a disposable bandage contact lens is inserted and topical antibiotics instilled. Patients are evaluated post-operatively for 1 to 4 days after surgery and maintained on antibiotic treatment for appropriate periods of time (up to 1 month). Follow up examinations performed at 1, 3 and 6 months include visual acuity testing, manifest refraction, slit lamp evaluation and in vivo confocal microscopy.

### Example 3

### Gene Therapy Methods

Next are described methods to convert adipose tissue-derived stromal cells into cells expressing at least one growth factor or protein accelerating intra-ocular repair (TGFβ blocking protein) using any vector approach (viral, transfection, other). Surgical patients undergo phototherapeutic keratectomy using a 193 nm excimer beam generated within a VISX Star S2 Excimer Laser (VISX, Inc., Santa Clara, CA) and delivered at 10 Hz with a fluence of 160 mJ/cm² as described in Lee et al (2001, Ophthalmology; 108(1):112-20). A standardized, central 6-mm diameter laser photoablation is performed with a 45 µm depth setting for epithelial debridement and an appropriate setting for stromal ablation. After PRK, either undifferentiated or adipocyte differentiated adipose tissue-derived adult stem cells are introduced into the ablated area in a composition with a biocompatible material. Prior to introduction, the cells are transfected or transduced with an appropriate nucleic acid vector expressing a soluble type II transforming growth factor beta receptor protein. This protein binds to the transforming growth factor beta cytokine and inhibits its ability to initiation a signal transduction cascade at the cellular level (Rowland-Goldsmith et al 2001, Clin Cancer Res. 2001, 7(9):2931-40). Transforming growth factor beta is known to interfere with recovery following corneal surgery and promotes corneal fibrosis (Jester et al 1997, Cornea; 16(2):177-87).

The genetically engineered cells are then cultured in a directional manner on the surface of a biocompatible membrane, including but not limited to, amniotic membrane, swine intestinal submucosa, Ampligraft^{™}, Dermagraft^{™}, or a similar product. The cells are cultured as either undifferentiated fibroblast-like cells or induced to undergo adipocyte differentiation according to the methods described in Halvorsen et al (2001, Metabolism 50:407-413). The cell/biomaterial composite is cut to fit the defect size. The cell surface is placed adjacent to the denuded area of cornea. The composite of cells/biomaterial is sutured into place using 10-0 nylon using interrupted or continuous bites in the cornea and limbus; any excess composite is trimmed away (Anderson et al 2001, Br J Ophthalmol; 85(5):567-575). Following the surgery, a disposable bandage contact lens is inserted and topical antibiotics instilled.

Patients are evaluated post-operatively for 1 to 4 days after surgery and maintained on antibiotic treatment for appropriate periods of time (up to 1 month). Follow up examinations performed at 1, 3 and 6 months include visual acuity testing, manifest refraction, slit lamp evaluation and in vivo confocal microscopy.

### Example 4

### Simple Transplantation

Adipose tissue-derived stromal cells are transplanted alone intra-ocularly. Surgical patients undergo phototherapeutic keratectomy using a 193 nm excimer beam generated within a VISX Star S2 Excimer Laser (VISX, Inc., Santa Clara, CA) and delivered at 10 Hz with a fluence of 160 mJ/cm² as described in Lee et al (2001, Ophthalmology; 108(1):112-20). A standardized, central 6-mm diameter laser photoablation is performed with a 45 µm depth setting for epithelial debridement and an appropriate setting for stromal ablation. After PRK, either undifferentiated or adipocyte differentiated adipose tissue-derived adult stem cells are introduced into the ablated area by direct injection. The cells are introduced as either a single cell suspension or as sheets of cells. Following the surgery, a disposable bandage contact lens is inserted and topical antibiotics instilled. Patients are evaluated post-operatively for 1 to 4 days after surgery and maintained on antibiotic treatment for appropriate periods of time (up to 1 month). Follow up examinations performed at 1, 3 and 6 months include visual acuity testing, manifest refraction, slit lamp evaluation and in vivo confocal microscopy.

### Example 5

### Cytokine Expression Profile of Human Adipose-Derived Stromal Cells

The cytokine expression profile of human adipose derived stromal cells from multiple donors has been determined. In these experiments, confluent, quiescent adipose derived stromal cell cultures were induced with lipopolysaccharide (LPS, 100 ng/ml) and conditioned medium and total RNA were harvested after periods of 1 to 24 hours. In common with both murine and human bone marrow-derived stromal cells, adipose derived stromal cells expressed the following cytokine mRNAs: interleukins 6, 7, 8, and 11 (IL-6,-7,-8,-11), leukemia inhibitory factor (LIF), macrophage-colony stimulating factor (M-CSF), granulocyte-macrophage-colony stimulating factor (GM-CSF), granulocyte-colony stimulating factor (G-CSF), flt-3 ligand, stem cell factor, tumor necrosis factor α (TNFα) and bone morphogenetic proteins 2 and 4 t (BMP-2, -4).

### Example 6

### Ability of Human Adipose-Derived Stromal Cells To Support The Proliferation And Differentiation of Human Umbilical Cord Blood Progenitor Cells

The ability of human adipose-derived stromal cells to support the proliferation and differentiation of human umbilical cord blood CD34⁺ hematopoietic progenitor cells in co-cultures was determined. Confluent cultures of adipose-derived stromal cells were established in 24 well plates (6 X 10⁴ cells per well). Umbilical cord blood (UCB) specimens were depleted of contaminating erythrocytes by treatment with hetastarch and of contaminating granulocytes by Ficoll density centrifugation. The remaining UCB mononuclear cells were lineage depleted according to the *StemSep*^{™} (StemCells, Vancouver, BC) protocol; this relies on immunomagnetic negative cell selection using a cocktail of antibodies directed against CD2, CD3, CD14, CD16, CD19, CD24, CD56, CD66b, and glycophorin A. In the last purification step, the *lin⁻* UCB cells were stained with CD34 antibodies and sorted by flow cytometry. Up to 10,000 of the final CD34⁺ UCB cells have been co-cultured in individual wells with a confluent adipose-derived stromal cell layer. Cultures were maintained in the absence of exogenous cytokines for periods of 12-days, 3 weeks, or 6 weeks. At the end of these periods, individual wells were harvested by trypsin/EDTA digestion and analyzed by flow cytometry using a combination of the following antibody combinations (fluorescent tags indicated in parentheses): CD45 (FITC), CD34 (APC), and either CD7, CD10, or CD38 (PE). The results of these assays are described below.

These studies examined the expansion of UCB hematopoietic cell populations in 12-day adipose stromal-supported co-cultures. In the absence of exogenous cytokines, adipose-derived stromal cells supported a 5.1 -fold expansion of total hematopoietic cell numbers (average, n = 4 stromal donors, n = 2 UCB donors; range 2 - 9.4). This corresponded to a 2.4- fold expansion of the CD34⁺ UCB cell population (average, n = 4 stromal donors, n = 2 UCB donors; range 1.4 - 3.3). A significant percentage of both the CD34⁺ and CD34' cells expressed either the CD7 or CD10 antigen (Figure 4, average, n = 4 stromal donors, n = 2 UCB donors). The individual phenotypes represented the following percentages of the total hematopoietic population: early lymphoid progenitors, 20.2% (CD34⁺ CD7⁺⁾ and 9.5% (CD34⁺ CD10⁺), respectively; NK/T-cell progenitors (CD34⁻ CD7⁺), 31.4%; and B-cell progenitors (CD34⁻ CD10⁺), 7.7%.

Further analysis indicated that there was a significant expansion of the early lymphoid progenitors. The CD34⁺ CD7⁺ population was expanded 4.8 ± 2.2-fold over the 12-day period progenitors (mean ± s.d., n = 4 stromal donors, n = 2 UCB donors). Likewise, the CD34⁺ CD10⁺ population was expanded 3.5 ± 1.6-fold progenitors (mean ± s.d., n = 4 stromal donors, n = 2 UCB donors). These values exceed the -fold expansion of the CD34⁺ population overall and suggests that this approach will enrich human lymphoid progenitors. These results indicate that the adipose-derived stromal cells can support the differentiation of hematopoietic progenitor cells in vitro.

Modifications and other embodiments of the invention will be apparent to one skilled in the art to which this invention pertains having the benefit of the teachings presented in the foregoing descriptions and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. An isolated adipose tissue-derived stromal cell induced to express at least one characteristic of an ocular cell.

2. The ocular cell of claim 1 differentiated in vitro.

3. The ocular cell of claim 1 differentiated in vivo.

4. The ocular cell of claim 1, wherein exogenous genetic material has been introduced into the isolated adipose tissue-derived stromal cell prior to differentiation.

5. The ocular cell of claim 1, wherein the cell is human.

6. The ocular cell of claim 1 implanted into a host.

7. The ocular cell of claim 1, wherein exogenous genetic material has been introduced into the cell.

8. The ocular cell of any of claims 1-8, wherein the cell is a corneal epithelial cell.

9. The ocular cell of any of claims 1-8, wherein the cell is an ocular stromal cell.

10. A composition comprising an isolated adipose tissue-derived stromal cell induced to express of at least one characteristic of an ocular cell and a biocompatible material.

11. The composition of claim 10, wherein the biocompatible material is selected from the group consisting of amniotic membrane, collagen, hydrogel, polyglycolic acid, polylactic acid, polyglycolic/polylactic acid, hyaluronate, or fibrin.

12. The composition of claim 11, wherein the biocompatible material is amniotic membrane.

13. The composition of any of claims 10-12, wherein the ocular cell is a corneal epithelial cell.

14. The composition of any of claims 10-12, wherein the ocular cell is an ocular stromal cell.

15. A method for differentiating isolated adipose tissue-derived stromal cells to display at least one ocular-associated cell marker comprising the step of contacting an isolated adipose tissue-derived stromal cell with an intra-ocular tissue site in a host.

16. The method of claim 15, wherein the isolated adipose tissue-derived stromal cells are obtained from the host.

17. The method of claim 15, wherein the adipose tissue-derived stromal cells are isolated from a donor that is not the host.

18. A method for differentiating isolated adipose tissue-derived stromal cells to display at least one ocular cell marker comprising contacting an isolated adipose tissue-derived stromal cell with an ocular inducing substance.

19. The method of claim 18, wherein the ocular inducing substance is in a chemically defined cell culture medium.

20. The method of claim 18, wherein the ocular cell marker is a marker for a corneal epithelial cell.

21. The method of claim 18, wherein the ocular cell marker is a marker for an ocular stromal cell.

22. A method of treating an ocular disease, degenerative condition or post-surgical condition in a host comprising:
i) inducing an isolated adipose tissue-derived stromal cell to express at least one ocular cell marker; and
ii) transplanting the induced cell into the host.

23. The method of claim 22, wherein the adipose tissue-derived stromal cells are isolated from the host.

24. The method of claim 22, wherein the ocular disorder, degenerative condition or surgical condition is aniridia, eythrokeratodermia, keratitis, chemical injuries; thermal injuries; contact lens keratopathy; repetitive limbal surgeries, limbal insufficiency, photorefractive keratectomy, laser in situ keratomileusis, an autoimmune dysfunction, or viral or bacterial infection.

25. A method of treating an ocular disease, degenerative condition or post surgical condition in a host comprising
i) isolating an adipose tissue-derived stromal cell; and
ii) transplanting the cell into an intra-ocular site in the host.

26. The method of claim 25, wherein the adipose tissue-derived stromal cells are isolated from the host.

27. The method of claim 25, wherein the ocular disorder, degenerative condition or surgical condition is aniridia, eythrokeratodermia, keratitis, chemical injuries; thermal injuries; contact lens keratopathy; repetitive limbal surgeries, limbal insufficiency, photorefractive keratectomy, laser in situ keratomileusis, an autoimmune dysfunction, or viral or bacterial infection.

28. The method of any of claims 25-27, wherein the adipose tissue-derived stromal cell is de-differentiated prior to transplantation into the host.
